# EUROPÄISCHE PATENTSCHRIFT

(11) **EP 3 890 955 B1**
(45) Veröffentlichungstag und Bekanntmachung des Hinweises auf die Patenterteilung: **10.07.2024**
(21) Anmeldenummer: 19821255.7
(22) Anmeldetag: 03.12.2019
(51) Int. Cl.: B30B 5/06, B27N 3/24, B30B 15/26, B27N 1/02, B27N 3/14, B32B 37/10, G01N 23/083, G01N 9/00, G01N 33/46

(54) **KONTINUIERLICH ARBEITENDE PRESSE MIT ROHDICHTEPROFILREGELUNG**
CONTINUOUSLY OPERATING PRESS HAVING CLOSED-LOOP RAW DENSITY PROFILE CONTROL
PRESSE FONCTIONNANT EN CONTINU À RÉGULATION DE PROFIL DE DENSITÉ APPARENTE

(30) Priorität: 06.12.2018 DE 102018131159
(43) Veröffentlichungstag der Anmeldung: 13.10.2021
(73) Patentinhaber: DIEFFENBACHER GMBH MASCHINEN- UND ANLAGENBAU, 75031 Eppingen (DE)
(72) Erfinder: HEMPEL, Tobias, 75031 Eppingen (DE)
(74) Vertreter: Hartdegen, Helmut
(86) Internationale Anmeldenummer: PCT/EP2019/083483
(87) Internationale Veröffentlichungsnummer: WO 2020/115038

(56) Entgegenhaltungen:
- EP-A1- 2 527 113
- WO-A1-2018/046390
- DE-A1- 10 123 793
- DE-A1- 10 231 031
- DE-C1- 3 907 617
- DE-T2- 69 801 228

## Beschreibung

Die Erfindung betrifft eine kontinuierlich arbeitende Presse nach dem Oberbegriff des Patentanspruchs 1.

Bei der industriellen Herstellung von Holzwerkstoffplatten kommen in der Regel kontinuierlich arbeitende Pressen zum Einsatz. Bei diesen Pressen, wie in DE 39 13 991 C2 beschrieben, wird die Presskraft durch hydraulische Stellglieder, wie insbesondere hydraulische Druckzylinder, auf die Press- und Heizplatten und weiter über Stahlbänder, die abgestützt über einen Wälzkörperteppich (Rollstangen) umlaufend angeordnet sind, auf das Pressgut übertragen. Der Kraftfluss für die in den hydraulischen Stellgliedern erzeugten Kräfte wird im Wesentlichen durch die Pressengestelle geleitet und abgestützt. Weitere Fortentwicklungen derartiger Pressen sind in DE 40 17 791 C2, DE 43 40 982 B4 und WO 2016/180899 A1 zu finden.

Zu Zwecken der Qualitätskontrolle ist es im Stand der Technik weiter üblich, an dem verpressten Plattenstrang Laborschnitte vorzunehmen, welche in einem Labor untersucht werden, um deren Rochdichteprofil zu ermitteln. Basierend auf den so ermittelten Ergebnissen kann die Einhaltung der gewünschten Produkteigenschaften, wie Zug- und Biegefestigkeiten, Oberflächengüte (Optische Oberflächenqualität, Tieffräsfähigkeit, Oberflächenhärte etc.) überprüft und überwacht werden.

Die so erhaltenen Ergebnisse, insbesondere ein Vergleich des Soll-Dichteprofils mit dem gemessenen Dichteprofil, werden regelmäßig auch dem Produktionsleiter, Technologen und/oder dem Pressenbediener bereitgestellt, damit dieser ausgehend davon und basierend auf seinem Erfahrungsschatz händisch die Einstellungen der Presse, insbesondere der von den hydraulischen Stellgliedern aufgebrachten Pressdrücke, auch als Presskurve bezeichnet, so anpassen kann, dass sich das Ist-Rohdichteprofil dem gewünschten Soll-Rohdichteprofil annähert, bzw. idealer Weise diesem entspricht. Eine derartige Nachjustierung der Presseneinstellungen, und insbesondere der Presskurve, kann wie allgemein insbesondere dann notwendig werden, wenn beispielsweise sich die Art oder Feuchtigkeit des zur Span- oder Faserherstellung verwendeten Holzmaterials ändert, Parameter der Zerfaserung oder Beleimung geändert werden, die Pressengeschwindigkeit erhöht oder erniedrigt wird, etc.

Nachteilig hierbei ist, dass das Ist-Rohdichteprofil nur für einzelne Zeitpunkte erstellt wird und erst mit großer zeitlicher Verzögerung zur Verfügung steht. Im Falle sich ändernder Prozessparameter, wie beispielsweise sich ändernden Feuchte des verwendeten Holzmaterials, und damit einhergehender sich ändernder Ist-Rohdichte des Plattenstrangs, kann daher der Produktionsleiter, der Technologe und/oder dem Pressenbediener nur mit einer relativ langen Verzögerung reagieren und entsprechende Änderungen an den Presseneinstellungen vornehmen.

Diese große zeitliche Verzögerung kann bedeuten, dass für einen entsprechend langen Zeitraum Platten mit einem nicht die Qualität gewünschten und erforderlichen Dichteprofil gefertigt werden und daher Ausschuss produziert wird. Diese Problematik besteht insbesondere für im Markt zunehmend nachgefragte mehrschichtige Werkstoffplatten mit variablen Dichten, wie sie beispielsweise aus der WO 2005/046950 A1 bekannt sind. Diese Werkstoffplatten zeichnen sich insbesondere dadurch aus, dass zur Materialeinsparung, und damit einhergehend zur Gewichts- und Kostenersparnis, vorgesehen ist, die Platten Deckschichten mit vergleichsweiser hoher Dichte und eine Zwischenschicht mit variabler und vergleichsweise kleiner Dichte aufweisen. Wie leicht verständlich, ist es auch für die Fertigung derartiger Werkstoffplatten von großer Wichtigkeit, dass ein gewünschtes Soll-Rohdichteprofil mit hoher Genauigkeit eingehalten wird.

Weiter ist die DE 101 23 793 A1 mit einer Regelung bekannt geworden, bei der anhand der Ansteuerung der Druckelemente das Abstandsprofil der Pressplatten quer zur Laufrichtung, ggfs. unter Verbiegung der Pressplatten, eingesteuert werden kann. Hier zur wird das Eigenschaftsprofil der Werkstoffplatte nach der Presse quer zur Laufrichtung vermessen.

Auch die WO 2018/046390A1 thematisiert die Regelung einer kontinuierlichen Presse, in dem die Temperatur der Werkstoffplatte, ggfs. quer zur Laufrichtung, nach der Presse vermessen wird, um unter anderem die Geschwindigkeit oder die Heizleistung zu regeln.

Um diese Problematik zu adressieren, ist es ebenfalls bekannt, Messgeräte zur Bestimmung der Rohdichte in unmittelbarer Nähe der Presse anzuordnen, um eine kontinuierliche und direkte Erfassung der Ist-Rohdichte des von der Presse abgegebenen Plattenstranges zu erfassen. Derartige Messgeräte arbeiten berührungslos mittels Röntgenstrahlung, wobei eine Röntgenquelle Röntgenstrahlung in einem Winkel durch den Plattenstrang sendet, und wobei auf der entgegengesetzten Seite des Plattenstranges angeordnete Detektoren die transmittierte Strahlung und die von dem Plattenstrang gestreute Strahlung erfassen, um so die Dichte des Plattenstrangs zu erfassen. Ein entsprechendes Messgerät wird beispielsweise von der Firma Fagus-GreCon Greten GmbH & Co. KG, 31061 Alfeld, Deutschland, mit der Bezeichnung Stenograph hergestellt.

Es wird somit eine Inline-Bestimmung der Ist-Rohdichte des Plattenstrangs direkt an der Presse selbst ermöglicht, so dass dem Pressenbediener die entsprechende Information jederzeit zur Verfügung steht und eine entsprechend rasche Reaktion grundsätzlich ermöglicht wird. Insbesondere erhält ein Pressenbediener, wenn er Veränderungen an den Presseneinstellungen, insbesondere an der Presskurve, vornimmt, eine sehr rasche Rückmeldung darüber, wie sich die vorgenommenen Einstellungsänderungen auf die Ist-Rohdichte auswirken, und kann daher, basierend auf seinen Erfahrungen und mit einem gewissen Maß an Trial-and-Error bzw. Versuch und Irrtum, rascher zu einer guten Presseneinstellung gelangen.

Auch wenn auf diese Weise die Arbeit des Pressenbedieners besser unterstützt wird, besteht jedoch weiterhin der Nachteil, dass es wesentlich von dem Wissen und Erfahrungsschatz des Pressenbedieners abhängt, entsprechend gute Presseneinstellungen zu finden und vorzunehmen. Dies erfordert zum einen den Einsatz von entsprechend gut ausgebildetem und qualifiziertem Personal als Pressenbediener, was mit entsprechenden Lohnkosten verbunden ist. Zum anderen ist auch ein gut ausgebildeter und erfahrener Pressenbediener oftmals zwar in der Lage, gute Presseneinstellungen aufzufinden und vorzunehmen, die eine hinreichende Qualität und Güte der hergestellten Platten sicherstellen, wobei jedoch weiterhin Optimierungsmöglichkeiten hinsichtlich nochmals besserer Presseneinstellungen ungenutzt bleiben.

Darüber hinaus erfordert eine (weitgehend) kontinuierliche Überwachung der Presse hinsichtlich des Ist-Rohdichteprofils des von der Presse hergestellten Plattenstrangs auch die (weitgehend) kontinuierliche Aufmerksamkeit des Pressenbedieners, so dass dieser nur in begrenztem Maße andere Aufgaben wahrnehmen kann, welche anderen Aufgaben entsprechend von anderem Personal erfüllt werden müssen.

Es ist daher eine Aufgabe der vorliegenden Erfindung, eine Lösung anzugeben, welche die vorstehenden Nachteile überwindet.

Insbesondere ist es eine Aufgabe der vorliegenden Erfindung, eine kontinuierlich arbeitende Presse anzugeben, die eine bessere Optimierung ermöglicht und/oder einen Pressenbediener in seiner Arbeitsbelastung entlastet.

Diese und andere Aufgaben der vorliegenden Erfindung werden gelöst von einer kontinuierlich arbeitenden Presse wie in Anspruch 1 angegeben. Weitere bevorzugte Ausführungsformen sind in den abhängigen Ansprüchen dargelegt.

Als eine Lösung wird eine kontinuierlich arbeitende Presse angegeben, umfassend: zwei endlos umlaufende, Pressgut durch die Presse ziehende Stahlbänder, die zwischen sich zur Verpressung des Pressgutes einen Presspalt ausbilden, die Stahlbänder abstützende temperierbare Heizplatten, eine zwischen den Stahlbändern und den Heizplatten angeordnete Wälzlagerabstützung, mehrere quer zur Umlaufrichtung der Stahlbänder angeordnete Gestelle zur Aufnahme der Presskräfte, die über mehrere in einer Reihe angeordnete hydraulische Arbeitszylinder je Gestell zur Verpressung des Pressguts zu einer Werkstoffplatte und zur Einstellung des Pressspaltes direkt in die Heizplatte eingeleitet werden, und ein Steuersystem zur Steuerung und/oder Regelung des Betriebs der Presse, wobei weiter eine Messeinrichtung zur Erfassung des Rohdichteprofils der Werkstoffplatte vorgesehen ist, das Steuersystem eingerichtet ist, die Arbeitszylinder gemäß einer Presskurve zu steuern und/oder zu regeln, und das Steuersystem eine Analysevorrichtung umfasst, welche die von der Messeinrichtung erfassten Messwerte empfängt, das erfasste Rohdichteprofil als ein Ist-Rohdichteprofil, hinsichtlich Abweichungen zu einem Soll-Rohdichteprofil analysiert, und basierend auf der Analyse an der Presskurve vorzunehmende Änderungen bestimmt, um das Ist-Rohdichteprofil dem Soll-Rohdichteprofil anzunähern oder anzugleichen.

Die von der Analysevorrichtung bestimmten an der Presskurve vorzunehmenden Änderungen können vorzugsweise als Empfehlungen auf einer Anzeige- und Bedienvorrichtung angezeigt werden, so dass ein Pressenbediener der Presse entsprechende Einstellungsänderungen vornehmen kann. Alternativ oder ergänzend kann das Steuersystem die Presskurve automatisch gemäß den von der Analysevorrichtung bestimmten an der Presskurve vorzunehmenden Änderungen anpassen.

Es wird somit eine semi-automatische oder automatische Rohdichteprofilregelung verwirklicht, welche eine Optimierung des Pressenbetriebs, und damit eine Verbesserung der Qualität und Güte, insbesondere hinsichtlich kontinuierlich guter mechanischer Platteneigenschaften, und damit einer höheren Wertigkeit, der hergestellten Werkstoffplatten ermöglicht. Gleichzeitig wird der Ausschuss vermindert. Dies gilt insbesondere, da die guten mechanischen Platteneigenschaften zu einer verbesserten Tieffräsbarkeit und damit zu verbesserten Verarbeitungsmöglichketen führen, mit ebenfalls entsprechend gesenktem Plattenausschuss.

Die erzielbare kontinuierlich hohe Qualität und Güte der Herstellung ermöglicht auch, die Gesamtrohdichte der Werkstoffplatten zu senken, was zu entsprechenden Materialersparnissen und damit besserer Wirtschaftlichkeit führt.

Gleichzeitig wird es ermöglicht, einen Pressenbediener ganz oder teilweise von der Aufgabe der Überwachung der Presse und der Einstellung und Anpassung der Presse an sich ändernde Gegebenheiten entlastet. Der Pressenbediener kann sich so (auch) anderen Aufgaben widmen, was zu einem verbesserten Personaleinsatz und damit verbesserter Wirtschaftlichkeit führt.

Bevorzugt kann die Analysevorrichtung das Ist-Rohdichteprofil in eine Mehrzahl von Bereichen unterteilen, wobei sich jeweils benachbarte Bereiche teilweise überlappen, und wobei die Analyse des Ist-Rohdichteprofils hinsichtlich der Abweichungen zu dem Soll-Rohdichteprofil bereichsweise erfolgt. Insbesondere kann das Ist-Rohdichteprofil und/oder das Soll-Rohdichteprofil in zumindest fünf Bereiche unterteilt werden, wobei die Bereiche zwei Außenbereichen, zwei Übergangsbereichen und einem Kernbereich entsprechen.

In einer bevorzugten Ausführungsform wird das Ist-Rohdichteprofil in neun Bereiche unterteilt, wobei die beiden Außenbereiche und Übergangsbereiche in je zwei Bereiche unterteilt werden. Hierdurch ist eine noch bessere Analyse des Ist-Rohdichteprofils möglich.

Alternativ oder ergänzend umfasst der Außenbereich den Bereich mit einer mittleren Dichte größer 100%, bevorzugt größer 105% und/oder der Übergangsbereich einen Bereich von 88% bis 110%, bevorzugt von 90% bis 108% der mittleren Dichte und/oder der Kernbereich einen Bereich von 75% bis 95%, bevorzugt von 80% bis 93% der mittleren Dichte.

Es kann vorgesehen sein, dass die Analysevorrichtung als eine Fuzzy-Regeleinheit ausgebildet ist oder diese umfasst.

Alternativ oder ergänzend kann vorgesehen sein, dass die Analysevorrichtung als ein neuronales Netz, insbesondere ein Deep Learning-neuronales Netz ausgebildet ist oder dieses umfasst.

Es kann auch vorgesehen sein, dass die Analysevorrichtung als Eingabe zusätzlich zumindest einen und bevorzugt alle der folgenden weiteren Parameter und/oder Messwerte berücksichtigt: Holzart, Faserlänge, Faserqualität, Kochzeit, Kochdruck, Mahlscheiben-Distanz, Bindemitteltyp, Bindemittelart, Bindemittelmenge, Härtertyp, Härterart, Härtermenge. Blasventilposition, Materialfeuchte, Schüttdichte des Materials, Fasertemperatur, Vorpressendruck, Pressfaktor der Presse, Mattenbefeuchtung / Besprühung Matten-Flächengewicht, Mattenbreite, Mattentemperatur, Heizplattentemperatur, und Pressengeschwindigkeit.

Es kann ebenfalls vorgesehen sein, dass das Steuersystem weiter eingerichtet ist, kontinuierlich oder in regelmäßigen Abständen die gemessenen Ist-Rohdichteprofile, die aktuelle Presskurve, sowie zusätzlich zumindest einen und bevorzugt alle der folgenden weiteren Parameter und/oder Messwerte zu berücksichtigen: Holzart, Faserlänge, Faserqualität, Kochzeit, Kochdruck, Mahlscheiben-Distanz, Bindemitteltyp, Bindemittelart, Bindemittelmenge, Härtertyp, Härterart, Härtermenge. Blasventilposition, Materialfeuchte, Schüttdichte des Materials, Fasertemperatur, Vorpressendruck, Pressfaktor der Presse, Mattenbefeuchtung / Besprühung Matten-Flächengewicht, Mattenbreite, Mattentemperatur, Heizplattentemperatur, und Pressengeschwindigkeit.

Alternativ oder ergänzend kann es auch vorgesehen sein, dass die Analysevorrichtung als Eingabe zusätzlich zumindest einen und bevorzugt alle der folgenden weiteren Parameter und/oder Messwerte berücksichtigt: Partikelgröße, Partikelgrößenverteilung, Art der Zerkleinerungsvorrichtung, Standzeit der Zerkleinerungsvorrichtung und Schleifzugabe.

Alternativ oder ergänzend kann es auch vorgesehen sein, dass das Steuersystem weiter eingerichtet ist, kontinuierlich oder in regelmäßigen Abständen die gemessene Ist-Rohdichteprofile, die aktuelle Presskurve, sowie zusätzlich zumindest einen und bevorzugt alle der folgenden weiteren Parameter und/oder Messwerte berücksichtigt: Partikelgröße, Partikelgrößenverteilung, Art der Zerkleinerungsvorrichtung, Standzeit der Zerkleinerungsvorrichtung und Schleifzugabe. Weitere vorteilhafte Maßnahmen und Ausgestaltungen des Gegenstandes der Erfindung gehen aus der folgenden Beschreibung mit der Zeichnung hervor:
- Fig. 1: zeigt eine schematische Seitenansicht einer Presse gemäß einer Ausführungsform;
- Fig. 2: zeigt ein schematisches Schaubild eines Steuersystems der Presse der Fig. 1;
- Fig. 3: zeigt schematisch ein beispielhaftes Rohdichteprofil;
- Fig. 4: zeigt schematisch den Verlauf einer beispielhaften Presskurve;
- Fig. 5: zeigt schematisch eine Mehrzahl von Bereichen, in welchen das Rohdichteprofil der Fig. 3 analysiert wird;
- Fig. 6a bis 6d: zeigen schematisch beispielhafte unterschiedliche Rohdichteprofile für unterschiedliche Betriebssituationen.

In der Zeichnung wurde auf eine vollständige Abbildung einer kontinuierlich arbeitenden Presse verzichtet. Der bereits erläuterte und hier zugrunde liegende Stand der Technik ist dem Fachmann bekannt.

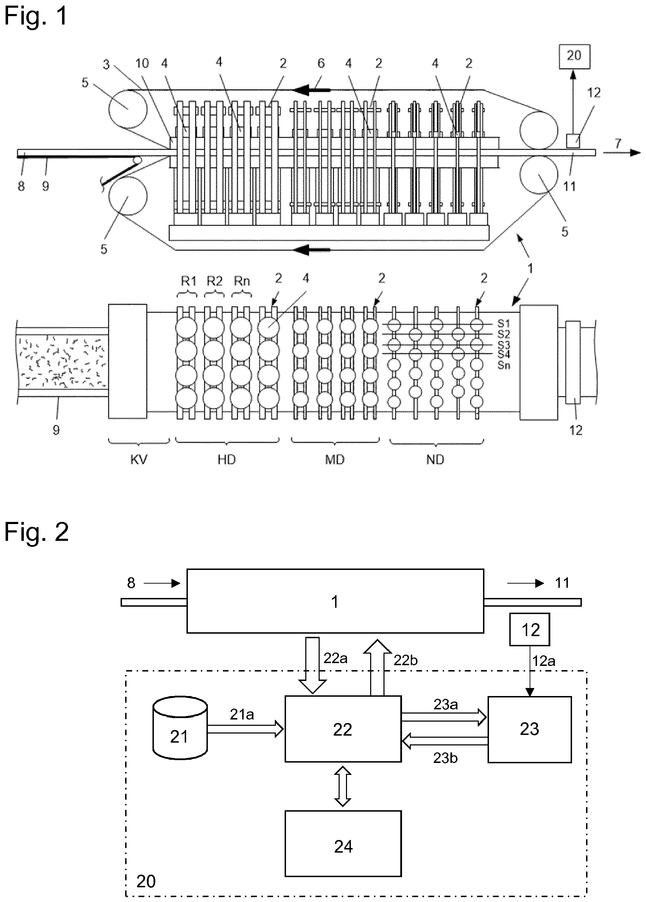

Die Fig. 1 zeigt oben eine schematische Seitenansicht einer Presse 1 mit zwei endlos umlaufenden Stahlbändern 3 und einer Produktionsrichtung 7 von links nach rechts. Die Stahlbänder 3 sind gegenläufig in Umlaufrichtung 6 um Umlenktrommeln 5 geführt und bilden zwischen sich einen Presspalt zur Verpressung von Pressgut 8 aus und ziehen dabei das Pressgut 8 durch die Presse 1. Die Stahlbänder 3 stützen sich über Wälzlager (nicht dargestellt) an den in Gestellen 2 gelagerten Heizplatten 10 ab, welche in diesem Ausführungsbeispiel von oben mit Arbeitszylindern 4 mit Druck beaufschlagt werden, wobei sich die Arbeitszylinder 4 wieder an den Gestellen 2 abstützen. In der unteren Draufsicht der Fig. 1 erkennt man den Keilverdichter KV einlaufseitig der Presse 1. Dieser übernimmt die Pressgutmatte 8 von einem Transportband 9und verdichtet sie gemäß einem voreingestellten Hauptverdichtungsprofil. Ist der Presspalt im Wesentlichen auf der Dicke des Endproduktes angekommen, befindet sich das Material im Hochdruckbereich (HD), nachfolgend erreicht es den Mitteldruckbereich (MD) und den Niederdruckbereich (ND). Die Durchwärmung und die Plastifizierung erfolgt im Wesentlichen in den ersten beiden Bereichen. Diese Bereiche weisen aufgrund ihrer hohen notwendigen Presskräfte im Wesentlichen massive Pressengestelle auf. Der Niederdruckbereich (ND) gibt der Werkstoffplatte 11 in der Regel Zeit fertig abzubinden. Im Sinne der Offenbarung wird dabei als Werkstoffplatte 11 sowohl der von der Presse 1 verpresste Plattenstrang, als auch ein einzelnes Plattenelement verstanden, das z.B. mittels einer der Presse 1 nachgelagerten Querschneidereinrichtung vom Plattenstrang abgetrennt wurde.

Der Presse 1 ist eine Messeinrichtung 12 nachgeschaltet, welche der Erfassung des Rohdichteprofils der Werkstoffplatte 11 dient. Die von der Messeinrichtung 12 ermittelten Messwerte werden an ein Steuersystem 20 übermittelt.

Der Aufbau und die Wirkungsweise des Steuersystems 20 wird mit Bezug auf Fig. 2 in größerem Detail beschrieben.

Wie in Fig. 2 gezeigt, beinhaltet das Steuersystem 20 eine Speichereinheit 21, eine Steuer- und/oder Regelvorrichtung 22, eine Analysevorrichtung 23 und eine mit der Steuer- und/oder Regelvorrichtung 22 verbundene Anzeige- und Bedienvorrichtung 24 auf.

In der Speichereinheit 21 sind verschiedene Programme, welche auch als Rezepte bezeichnet werden, für auf der Presse 1 herzustellende Werkstoffplatten 11 hinterlegt. In jedem dieser Rezepte sind dabei, als entsprechende Rezeptdaten, alle Daten betreffend der jeweils herzustellenden Werkstoffplatte 11 hinterlegt, wie etwa Daten von Referenzplatten, insbesondere Soll-Rohdichteprofil, Soll-Feuchtigkeit, Abmessungen in Länge, Dicke und Breite, sowie auch Referenz-Prozessparameter und Referenz-Presseneinstellungen, insbesondere eine Referenz-Presskurve. Ein Pressenbediener kann über die Anzeige- und Bedienvorrichtung 24 ein Rezept für eine herzustellende Werkstoffplatte 11 auswählen, worauf die Steuer- und/oder Regelvorrichtung 22 die entsprechende Rezeptdaten 21a aus der Speichervorrichtung 21 abruft, und die entsprechenden Parametereinstellungen der Presse 1 veranlasst, indem die Steuer- oder Regelvorrichtung 22 die entsprechenden Werte als Steuersignale bzw. Soll-Parameter 22b an die entsprechenden einzustellenden oder zu steuernden oder regelnden Komponenten der Presse 1 sendet, wie beispielsweise Soll-Druckwerte für die einzelnen hydraulischen Stellaggregate bzw. Arbeitszylinder 4 oder Soll-Temperatur(en) der Heizplatten 10. Die Steuer- oder Regelvorrichtung 22 empfängt weiter von der Presse 1, bzw. von den einzelnen Komponenten der Presse 1, Information über Messwerte, die von entsprechenden Sensoren erfasst werden, wie beispielsweise an den Arbeitszylindern vorgesehenen Drucksensoren zur Erfassung der Ist-Druckwerte, oder Temperatursensoren zur Erfassung der Temperatur(en) der Heizplatten 10 oder der Pressgutmatte 8. Mit diesen Werten führt die Steuer- und/oder Regelvorrichtung 22 auf grundsätzlich bekannte Weise eine Steuerung und/oder Regelung des Betriebs der Presse 1 aus.

Mit der Messeinrichtung 12 werden Messdaten bezüglich der Ist-Rohdichte der Werkstoffplatte 11 erfasst. Die Messung erfolgt dabei vorzugsweise berührungslos mittels Röntgenstrahlung, wobei eine Röntgenquelle Röntgenstrahlung in einem Winkel durch die Werkstoffplatte 11 sendet, und wobei auf der entgegengesetzten Seite der Werkstoffplatte 11 angeordnete Detektoren die transmittierte Strahlung und/oder die von der Werkstoffplatte 11 gestreute Strahlung erfassen, um so die Dichte der Werkstoffplatte 11 zu erfassen.

Die Messdaten werden an die Analysevorrichtung 23 weitergeleitet, welche aus den Messdaten ein Ist-Rohdichteprofil der Werkstoffplatte 11 ermittelt. Alternativ ist ebenso denkbar, dass die Verarbeitung der Messdaten und die Ermittlung des Ist-Rohdichteprofils bereits in der Messeinrichtung 12 selbst erfolgt und das ermittelte Ist-Rohdichteprofil an die Analysevorrichtung 23 gesendet wird. Das ermittelte Ist-Rohdichteprofil kann vorzugsweise auch auf der Bedien- und Anzeigevorrichtung 24 für einen Pressenbediener graphisch dargestellt werden.

Die Analysevorrichtung 23 ist weiter eingerichtet, das ermittelte Ist-Rohdichteprofil hinsichtlich Abweichungen von dem Soll-Rohdichteprofil zu analysieren, und basierend auf der Analyse weiter Änderungen an den Einstellungen Presse, und insbesondere Änderungen an der Presskurve, zu ermitteln, welche Änderungen bewirken, dass sich das Ist-Rohdichteprofil dem gewünschten Soll-Rohdichteprofil annähert, bzw. idealer Weise diesem entspricht. Zu diesem Zweck kann die Analysevorrichtung 23 von der Steuervorrichtung 22 das Soll-Rohdichteprofil, Daten zur aktuell eingestellten Presskurve und andere Daten als Pressen- und Rezeptbezogene Daten 23a abrufen.

Die so ermittelten Änderungen der Einstellungen, und insbesondere Änderungen an der Presskurve, werden von der Analysevorrichtung 23 als Einstellungsänderungsinformation 23b an die Steuervorrichtung 22 übermittelt. Die Steuervorrichtung 22 kann dann die von der Analysevorrichtung 23 ermittelten Einstellungsänderungen auf der Bedien- und Anzeigevorrichtung 24 für den Pressenbediener als einen Vorschlag, wie die Einstellungen abzuändern sind anzeigen, um etwaige Abweichungen des Ist-Rohdichteprofils vom Soll-Rohdichteprofil auszugleichen. Der Pressenbediener kann so selbst entscheiden, ob oder in welchem Maß er die vorgeschlagenen Einstellungsänderungen übernehmen möchte, und kann über die Anzeige- und Bedienvorrichtung 24 die entsprechenden, gewünschten Einstellungsänderungen vornehmen. Bevorzugt wird hierbei auf der Anzeige- und Bedienvorrichtung 24 die aktuell eingestellte Presskurve angezeigt, zusammen mit einer vorgeschlagenen abgeänderten Presskurveneinstellung. Es wird dem Pressenbediener auf diese Weise eine einfach verständliche Darstellung geboten, wie die Presskurve verändert werden soll, um das Ist-Rohdichteprofil dem Soll-Rohdichteprofil anzugleichen. Gleichzeitig erlaubt es diese Darstellung dem Pressenbediener auf rasche Weise die Plausibilität der vorgeschlagenen Einstellungsänderungen zu erkennen und nachzuvollziehen. Auf diese Weise wird eine semi-automatische Regelung des Rohdichteprofils erreicht, bei welcher der Pressenbediener davon befreit wird, die Analyse des Ist-Rohdichteprofildichte selbst vorzunehmen und basierend darauf zu bestimmen, wie und welche Einstellungen vorgenommen werden sollen, um etwaige Abweichungen des Ist-Rohdichteprofils von dem Soll-Rohdichteprofil auszugleichen. Dementsprechend wird es ermöglicht, dass die Presse 1 zum Beispiel (ganz oder teilweise) auch von weniger gut geschultem Personal bedient werden kann, während gleichzeitig eine sehr hohe Qualität und Güte der hergestellten Werkstoffplatten 11 ermöglicht wird. Darüber hinaus hat bei diesem semi-automatischen Betrieb der Pressenbediener weiterhin die volle Kontrolle über den Betrieb der Presse, was für eine bessere Akzeptanz dieser Regelung sorgen kann.

Alternativ und bevorzugt kann die Steuervorrichtung 22, basierend auf der von der Analysevorrichtung 23 übermittelten Einstellungsänderungsinformation 23b, die Einstellungsänderungen automatisch vornehmen, und kann insbesondere die Presskurve entsprechend anpassen, indem die jeweiligen Soll-Druckwerte für die einzelnen Arbeitszylinder 4 entsprechend abgeändert werden. Auf diese Weise bilden die Steuervorrichtung 22 und die Analysevorrichtung 23 zusammen mit der Presse einen geschlossenen Regelkreis für eine Rohdichteprofilregelung, das heißt, eine Regelung, welche die Abweichungen zwischen Ist-Rohdichteprofil und Soll-Rohdichteprofil minimiert. Eine derartige automatische Regelung der Rohprofildichte erlaubt einen automatischen Betrieb der Presse 1 bei sehr hohe Qualität und Güte der hergestellten Werkstoffplatten 11 und befreit den Pressenbediener der Presse 1 von der Notwendigkeit, den Betrieb der Presse 1 quasi kontinuierlich zu überwachen, so dass der Pressenbediener auch andere Aufgaben wahrnehmen kann.

Vorzugsweise kann auch vorgesehen sein, dass zwischen dem semi-automatischen und dem (vollständig) automatischen Betrieb umgeschaltet werden kann.

Es wird nun das Prinzip der Arbeitsweise der Analysevorrichtung 23 in größerem Detail beschrieben.

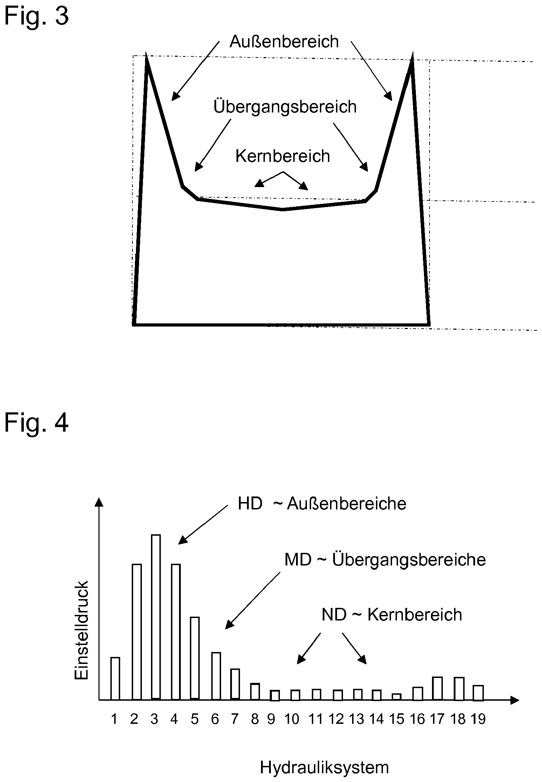

Die Fig. 3 zeigt den Verlauf eines ein beispielhaften Rohdichteprofils für eine Werkstoffplatte. Der Rohdichteprofilverlauf bezieht sich dabei auf den Verlauf der Rohdichte über die Plattendicke, das heißt, in Richtung einer Flächennormalen. Das dargestellte Rohdichteprofil weist in den Außenbereichen der Werkstoffplatte 11 eine höhere Dichte auf, welche jeweils mit einem raschen Abfall in einem Übergangsbereich übergehen in eine niedrigere Dichte des Zwischenbereichs. Das in Fig. 3 gezeigte Rohdichteprofil stellt dabei beispielhaft ein gewünschtes bzw. optimales Rohdichteprofil dar, das die herzustellenden Werkstoffplatten 11 einhalten sollen.

Die Fig. 4 zeigt eine entsprechende beispielhafte Presskurve, mit den entsprechenden Druckwerten für die (hier beispielhaft 19) Arbeitszylinder 4 entlang der Presse. Die Presskurve zeigt am Eingang der Presse im Hochdruckbereich HD (in Fig. 4 entsprechend etwa dem Bereich der Arbeitszylinder #1 bis #4), einen rasch ansteigendes Druckprofil, das über einen Bereich mittleren Drucks MD (in Fig. 4 entsprechend etwa dem Bereich der Arbeitszylinder #5 bis #8) in den Niederdruckbereich ND (in Fig. 4 entsprechend etwa dem Bereich beginnend ab Arbeitszylinder #9) übergeht. Am Ende der Pressstrecke steigt der Druck wiederum etwas an, um insbesondere im Rahmen einer Dickenregelung die Dicke der herzustellenden Werkstoffplatten 11 zu kontrollieren und zu regeln.

Aufgrund des Umstandes, dass die Durchwärmung des Pressgutstrangs, hervorgerufen von den Heizplatten 10, und die damit einhergehende Plastifizierung von außen nach innen erfolgt, ergibt sich ein gewisser Zusammenhang zwischen den sich einstellenden Profildichten in den Außenbereichen, den Übergangsbereichen und dem Kernbereich der herzustellenden Werkstoffplatten 11, einerseits, und den verschiedenen Bereichen der Presskurve. So wird die Dichte in den Außenbereichen in großem Maß von dem Verlauf der Presskurve am Eingangsbereich der Presse 1 bestimmt, mithin dem Hochdruckbereich HD (entsprechend etwa den Arbeitszylindern #1 bis #4 der Fig. 4), während der später plastifizierende Kernbereich in seiner Dichte eher von dem Verlauf der Presskurve im Niederdruckbereich ND (entsprechend etwa den Arbeitszylindern #8 bis #19 der Fig. 4) beeinflusst wird. Der dazwischen liegende Bereich der Presskurve, mithin der Bereich mittleren Drucks MD (entsprechend etwa den Arbeitszylindern #4 bis #8 der Fig. 4) beeinflusst entsprechend wesentlich die Übergangsbereiche zwischen den Außenbereichen und dem Kernbereich der herzustellenden Werkstoffplatte 11.

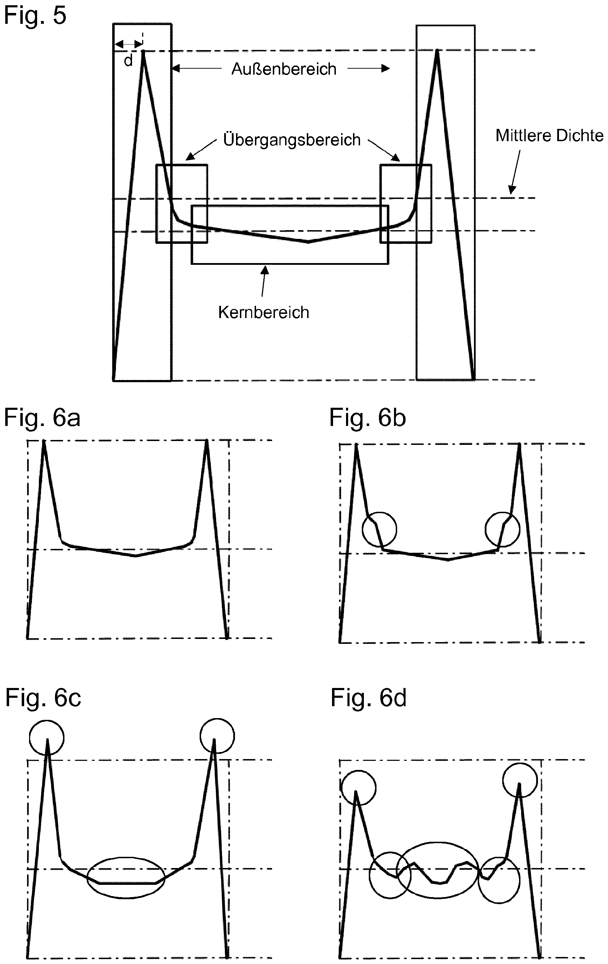

In einer einfachsten Form der Analysevorrichtung 23, gemäß einer ersten Ausführungsform, kann sich dieser Zusammenhang zunutze gemacht werden, indem die Analysevorrichtung 23 die Kurve des Ist-Rohdichteprofils bereichsweise analysiert. Dazu kann die Analysevorrichtung 23 das Ist-Rohdichteprofil in eine Mehrzahl von Bereichen unterteilen und diese Bereiche getrennt analysieren. Insbesondere kann die Analysevorrichtung 23, wie beispielhaft in Fig. 5 gezeigt, das Ist-Rohdichteprofil in fünf Bereiche unterteilen, entsprechend den zwei Außenbereichen, den zwei Übergangsbereichen und dem Kernbereich. Bevorzugt besteht dabei eine gewisse Überlappung zwischen jeweils zwei benachbarten Bereichen. Die Lage und Breite der jeweiligen Bereiche kann dabei vorzugsweise als Parameter in dem jeweiligen Rezept hinterlegt sein, so dass diese Bereiche für jede herzustellende Werkstoffplatte 11 geeignet vorgegeben werden können. Als eine geeignete Bemaßungsregel kann dabei auf den Abstand d des Maximums der Rohdichte in den Außenbereichen von den Plattenoberflächen zurückgegriffen werden. Mit diesem Abstand d als Basis können sich die Außenbereiche beispielsweise von den Plattenoberflächen jeweils über eine Breite von 1,5 d bis 2,0 d, vorzugsweise über eine Breite von 1,6 d bis 1,8 d, besonders bevorzugt über eine Breite von 1,7 d erstrecken. Die Übergangsbereiche können sich jeweils von einem Abstand von 1,2 d bis zu einem Abstand von 2,5 d von der entsprechenden Plattenoberfläche, bevorzugt von einem Abstand von 1,3 d bis zu einem Abstand von 2,4 d von der entsprechenden Plattenoberfläche, und besonders bevorzugt von einem Abstand von 1,5 d bis zu einem Abstand von 2,2 d von der entsprechenden Plattenoberfläche erstrecken. Der Kernbereich kann sich über den mittleren Bereich der Werkstoffplatte 11 erstrecken, bis zu einem Abstand von 2,0 d bis 2,8 d, bevorzugt bis zu einem Abstand von 2,2 d bis 2,5 d, besonders bevorzugt bis zu einem Abstand von 2,3 d von den Plattenoberflächen entfernt erstrecken.

Alternativ oder ergänzend kann die Bemaßungsregel aus Basis einer mittleren Dichte angegeben werden. Die mittlere Dichte ist dabei der Mittelwert der Dichte über das gesamte Rohdichte-Profil. Mit der mittleren Dichte als Basis kann sich der Kernbereich zwischen den Dichteprofilspitzen von 75% bis 95%, bevorzugt von 80% bis 93% der mittleren Dichte erstrecken. Der Übergangsbereich kann in einem Bereich von 88% bis 110%, bevorzugt von 90% bis 108% der mittleren Dichte angeordnet werden und der Außenbereich umfasst die Dichteprofilspitzen und liegt in einem Bereich > 100%, bevorzugt > 105% der mittleren Dichte.

In den Außenbereichen kann die Analysevorrichtung 23 beispielsweise die maximal erreichte Rohdichte, sowie die mittlere Rohdichte ermitteln. Im Vergleich mit den entsprechenden Werten, die sich aus dem gewünschten Soll-Rohdichteprofil ergeben, kann die Analysevorrichtung 23 so ermitteln, ob die Außenbereiche wie gewünscht verdichtet und ausgehärtet wurden. Alternativ oder ergänzend kann die Analysevorrichtung 23 für diese Bereiche auch ein Fehlermaß für die Abweichungen des Ist-Rohdichteprofils von dem Soll-Rohdichteprofil bestimmen, wie beispielsweise den mittleren Abweichungsfehler, die Summe der Quadrate der Abweichungen etc. Basierend auf diesen Daten kann die Analysevorrichtung 23 dann bestimmen, ob die Verdichtung in den Außenbereichen zu hoch oder zu gering ist, sowie das Ausmaß der Abweichung, und kann entsprechend bestimmen, dass eine Änderung der Einstellung der Solldruckwerte der Arbeitszylinder 4 im Hochdruckbereich HD vorgenommen werden soll, indem diese entsprechend angehoben oder abgesenkt werden.

Für die Übergangsbereiche kann die Analysevorrichtung 23 auf ähnliche Weise ein Fehlermaß für die Abweichungen des Ist-Rohdichteprofils von dem Soll-Rohdichteprofil bestimmen, wie beispielsweise den mittleren Abweichungsfehler, die Summe der Quadrate der Abweichungen etc. ermitteln, um basierend darauf zu bestimmen, ob und in welchem Ausmaß der Verlauf der Ist-Rohdichteprofilkurve in den Übergangsbereichen über oder unter dem entsprechenden Verlauf der Soll-Rohdichteprofilkurve liegt. Basierend darauf kann die Analysevorrichtung 23 dann entsprechend bestimmen, dass eine Änderung der Einstellung der Solldruckwerte der Arbeitszylinder 4 im Mitteldruckbereich MD vorgenommen werden soll, indem diese entsprechend angehoben oder abgesenkt werden.

Für den Kernbereich kann die Analysevorrichtung 23 beispielsweise die maximale und die minimale Rohdichte in diesem Bereich bestimmen und diese mit den entsprechenden Werten der Soll-Rohdichteprofilkurve vergleichen. Alternativ oder ergänzend können auch für den Kernbereich wiederum verschiedene Fehlermaße, wie beispielsweise den mittleren Abweichungsfehler, die Summe der Quadrate der Abweichungen etc. ermitteln, um basierend darauf zu bestimmen, ob und in welchem Ausmaß der Verlauf der Ist-Rohdichteprofilkurve im Kernbereich über oder unter dem entsprechenden Verlauf der Soll-Rohdichteprofilkurve liegt. Basierend darauf kann die Analysevorrichtung 23 dann entsprechend bestimmen, dass eine Änderung der Einstellung der Solldruckwerte der Arbeitszylinder 4 im Niederdruckbereich ND vorgenommen werden soll, indem diese entsprechend angehoben oder abgesenkt werden.

Diese Form einer Rohprofildichteregelung gemäß der ersten Ausführungsform eignet sich insbesondere, wenn die Presse 1 sehr nahe den Referenz-Betriebsparametern, für welche das Rezept erstellt wurde, betrieben werden kann, das heißt, nur kleine Abweichungen oder Schwankungen zum Beispiel der Span- oder Fasergüte des Pressgutmaterials vorliegen und dementsprechend nur geringe Abweichungen des Ist-Rohdichteprofils vom Soll-Rohdichteprofil vorliegen. Diese Form der Rohprofildichteregelung kann daher insbesondere im Fall einer Presse 1 verwendet werden, die bereits eine gute Einstellung der Pressen- und Betriebsparameter erfahren hat, um eine weitere Feineinstellung und damit Optimierung der Presskurve für einen weitgehend optimalen Betrieb der Presse 1, und damit eine Optimierung der Qualität der hergestellten Werkstoffplatten 11 zu sorgen.

Die vorstehend erläuterte Rohdichteprofilregelung gemäß der ersten Ausführungsform beschränkt sich allein auf eine bereichsweise Analyse und bereichsweise getrennte Einstellungsänderung bzw. Regelung beschränkt, bei der bereichsübergreifende, sich gegenseitig beeinflussende Wirkungen und/oder Einflüsse anderer Parameter unberücksichtigt bleiben. Für größere Abweichungen des Ist-Rohdichteprofils vom Soll-Rohdichteprofil, die sich beispielsweise bei stärkeren Schwankungen oder Abweichungen in der Art und/oder Feuchte des verwendeten Holzmaterials, den Parametern der Zerfaserung und/oder Beleimung, Änderungen der Pressengeschwindigkeit, der Temperatur der Heizplatten etc. ergeben können, ist es jedoch wünschenswert, und in einigen Fällen notwendig, auch den Einfluss dieser Parameter, sowie die Auswirkung von Änderungen an der Presskurve in einem Bereich auf alle Bereiche des Ist-Rohdichteprofils zu berücksichtigen.

Dies soll weiter mit Bezug auf die Fig. 6a bis 6d erläutert werden, welche verschiedene Beispiel von optimalen und nicht optimalen Rohdichteprofilen zeigen.

In der Fig. 6a ist zum Vergleich nochmals der Verlauf eines beispielhaften optimalen, gewünschten Rohdichteprofils dargestellt.

Im Vergleich dazu zeigt die Fig. 6b ein beispielhaftes Ist-Rohdichteprofil, in welchem die Übergangsbereiche jeweils Überhöhungen bzw. Höcker' aufweisen, welche in der Fig. 6b durch Kreise kenntlich gemacht sind. Dieser Verlauf des Ist-Rohdichteprofils zeigt an, dass in der Presse 1 nach der anfänglichen starken Pressung im Einlauf bzw. dem Bereich des Hochdrucks HD (entsprechend etwa den Arbeitszylindern #1 bis #4 der Fig. 4) der Druck im mittleren Druckbereich MD (entsprechend etwa den Arbeitszylindern #5 bis #7 der Fig. 4) nicht rasch genug abgesenkt wird. Dem kann dadurch begegnet werden, dass die Soll-Druckwerte der Arbeitszylinder 4 im Mitteldruckbereich MD entsprechend abgesenkt werden; eine Beeinflussung anderer Bereiche findet in diesem Fall nicht oder nur kaum statt, so dass für den Hochdruckbereich HD und den Niederdruckbereich ND keine Einstellungsänderungen vorzunehmen sind.

Fig. 6c zeigt den Fall eines beispielhaften Ist-Rohdichteprofils, in welchem die maximale Rohdichte in den Außenbereichen zu hoch ist bzw. die gewünschte Rohdichte überschreitet. Gleichzeitig verläuft die Rohdichte im Kernbereich zu niedrig, während in diesem Beispiel die Übergangsbereiche keine größeren Auffälligkeiten zeigen. Dies ist ein Anzeichen dafür, dass der Pressdruck im Hochdruckbereich HD zu hoch ist, während im Niederdruckbereich ND der Druck zu niedrig ist. Dementsprechend kann z.B. der Pressdruck der Arbeitszylinder 4 im Hochdruckbereich HD entsprechend abgesenkt und der Pressdruck der Arbeitszylinder 4 im Niederdruckbereich ND entsprechend angehoben werden.

Alternativ oder ergänzend kann dieses Rohdichteprofil auch so bewertet werden, dass dieser sich einstellende Verlauf (zumindest teilweise) das Ergebnis einer zu kleinen Pressengeschwindigkeit für ein Pressgut mit höher als erwarteter Feuchte ist. Dementsprechend kann - an Stelle von oder in Ergänzung zu einer Veränderung der Einstellung der Soll-Pressdrücke der Arbeitszylinder 4 - in diesem Beispiel auch die Pressengeschwindigkeit erhöht und somit die Produktionsleistung gesteigert werden.

Die Fig. 6d zeigt hingegen den Verlauf eines beispielhaften Ist-Rohdichteprofils, bei welchem die maximale Rohdichte in den Außenbereichen zu klein ist und die gewünschte maximale Rohdichte nicht erreicht. Im Kernbereich hingegen weist in diesem Beispiel eine stark schwankende Rohdichte auf, wobei auch in den Übergangsbereichen die Rohdichte einen Verlauf unterhalb des gewünschten Rohdichteverlaufs zeigen. Dieser Fall kann so bewertet werden, dass die Feuchtigkeit des Pressguts niedriger als erwartet ist und die dafür vorgesehene Pressengeschwindigkeit zu hoch ist. Darüber hinaus kann dieser Fall so bewertet werden, dass im Niederdruckbereich ND ein Druck über eine zu lange Strecke aufgebracht wird, so dass am Ende des Niederdruckbereichs ND noch ein Druck auf die bereits abgebundene und plastifizierte Werkstoffplatte 11 ausgeübt wird, unter welchem Druck die Bindungen in der Kernschicht der Werkstoffplatte 11 teilweise wieder aufgebrochen werden. Um dem entgegenzuwirken kann die Pressengeschwindigkeit abgesenkt und der Verlauf der Presskurve im Niederdruckbereich ND in der Länge gestaucht' und verkürzt werden.

In einer zweiten Ausführungsform können in der Analysevorrichtung 23 daher zusätzlich zu den Mitteln der Analyse und Bewertung der ersten Ausführungsform weitere Mittel vorgesehen werden, die auch eine bereichsübergreifende Analyse erlauben, und insbesondere weiter zur Analyse auch weitere Einstell- oder Betriebsparameter der Presse 1 verwenden vorgesehen werden, sowie auch weitere Rechenmittel, welche bereichsübergreifende Einstellungsänderungen ermitteln, um den Verlauf der gesamten Presskurve, sowie vorzugsweise ergänzend anderer Einstellparameter der Presse 1, wie insbesondere der Pressengeschwindigkeit möglichst optimal anpassen zu können. Dies kann insbesondere dadurch geschehen, dass die einzelnen Beeinflussungs- und Wirkungspfade mathematisch modelliert und in ein System gekoppelter Regelkreiselemente abgebildet werden.

Alternativ ist es auch möglich, die Analysevorrichtung 23 als eine Fuzzy-Regelvorrichtung auszuführen, mit einem Satz an Fuzzy-Regeln, welche von dem Hersteller der Presse 1 ermittelt und einprogrammiert werden und welche die Regeln zur Analyse und zur Bewertung des Ist-Rohdichteprofils bzw. der Abweichungen zum Soll-Rohdichteprofil, sowie die daraus abzuleitenden Schlüsse hinsichtlich vorzunehmender Einstellungsänderungen gemäß den Regeln der Fuzzy-Logik abbilden. Dieser Ansatz hat den Vorteil, dass sich auf diese Weise das bestehende, umfangreiche Wissen von hochqualifizierten Anlagenentwicklern und/oder Pressenbedienern auf relativ leichte Weise in eine maschinelle Regelung umsetzen lässt.

Weiter alternativ und besonders bevorzugt ist es jedoch auch möglich, die Analysevorrichtung 23 als ein neuronales Netzwerk, insbesondere als eine Deep Learning-neuronales Netzwerk auszubilden. Hierbei wird der Analysevorrichtung 23, bzw. den Eingängen des neuronalen Netzwerks vorzugsweise das Ist-Rohdichteprofil, das Soll-Rohdichteprofil, sowie eine große Anzahl an weiteren Einstell-, Mess- und Betriebsparametern der Presse 1 eingegeben, wobei das neuronale Netzwerk so trainiert ist, auf die Eingaben hin einen Satz an Einstellungsänderungen auszugeben zur Anpassung der Presskurve, und vorzugsweise weiterer Parameter der Presse 1, wie die Pressengeschwindigkeit, die Heizplattentemperatur, die Einstellungen zur Mattenbefeuchtung, etc., so dass die Einstellungsänderungen für eine Angleichung des Ist-Rohdichteprofils an das Soll-Rohdichteprofil sorgen.

Zur Erstellung oder Erweiterung einer Lernbasis zum Trainieren des neuronalen Netzwerkes kann dabei vorzugsweise vorgesehen sein, dass die Steuervorrichtung 22 während des Betriebs der Presse 1 im manuellen Modus ohne Rohdichteprofilregelung und/oder im semi-automatischen und/oder automatischen Betriebsmodus der Rohdichteprofilregelung die jeweiligen ermittelten Ist-Rohdichteprofile zusammen mit allen erfassbaren Einstell-, Mess- und Betriebsparametern aufzeichnet und in der Speichereinheit 21 abspeichert. Es kann auf diese Weise eine umfangreiche Lernbasis erhalten werden, welche eine Vielzahl von Betriebssituationen für eine Vielzahl an unterschiedlichen Rezepten bzw. herzustellenden Werkstoffplatten 11 und eine Vielzahl von eingesetzten Rohstoffen, wie unterschiedlichen Holztypen, Holzfeuchten, Bindemitteltypen, Bindemittelmengen, Presskurven etc. abbildet.

Diese Lernbasis kann dann dazu verwendet werden, um in einer ersten Lernphase ein erstes neuronales Netzwerk, als ein Streckenmodell, zu trainieren, welches das Verhalten der Presse 1 in den unterschiedlichen Betriebssituationen modelliert, und welches für die jeweilige Betriebssituation ein sich einstellendes, modelliertes Ist-Rohdichteprofil ausgibt.

Nachdem in der ersten Lernphase so ein Streckenmodell trainiert wurde, wird dieses fixiert, und es wird in einer zweiten Lernphase nun ein zweites neuronales Netzwerk, als das in der Analysevorrichtung 23 zu verwendendes neuronales Netzwerk, trainiert, unter Verwendung der Lernbasis und des ersten neuronalen Netzwerks als das Streckenmodell. Dabei wird dem nun zu lernenden zweiten neuronalen Netzwerk das von dem ersten neuronalen Netzwerk für eine Betriebssituation berechnete, modellierte Ist-Rohdichteprofil eingegeben, sowie die weiteren Parameter, wie Soll-Rohdichteprofil, etc., welche von dem zweiten künstlichen neuronalen Netz bei seinem Einsatz in der Analysevorrichtung 23 verarbeitet werden sollen. Die von dem zu trainierenden zweiten neuronalen Netzwerk ausgegebenen Werte der Einstellungsänderungen werden dann als Änderungen der Eingangswerte des ersten neuronalen Netzwerks als dem Streckenmodell übernommen. Das sich nun einstellende, vom ersten neuronalen Netzwerk als dem Streckenmodell berechnete, modellierte Ist-Rohdichteprofil wird mit dem Soll-Rohdichteprofil verglichen, und der sich ergebende Unterschied wird als Lernfehler für das weitere Trainieren des zweiten neuronalen Netzwerks verwendet.

Nach Abschluss der zweiten Lernphase kann das so trainierte zweite neuronale Netzwerk in der Analysevorrichtung 23 eingesetzt werden, um die gewünschte Rohdichteprofilregelung zu verwirklichen.

### Bezugszeichenliste P 1591 WO:

- 1: Presse
- 2: Gestell
- 3: Stahlband
- 4: Arbeitszylinder
- 5: Umlauftrommel
- 6: Umlaufrichtung
- 7: Produktionsrichtung
- 8: Pressgut
- 9: Transportband
- 10: Heizplatte
- 11: Werkstoffplatte
- 12: Messeinrichtung
- 20: Steuersystem
- 21: Speichereinheit
- 21a: Rezeptdaten
- 22: Steuer- oder Regelvorrichtung
- 22a: Ist-Parameter Presse
- 23b: Soll-Parameter Presse
- 23: Analysevorrichtung
- 23a: Pressen- und Rezeptbezogene Daten
- 23b: Einstellungsänderungsinformation
- 24: Anzeige- und Bedienvorrichtung
- KV: Keilverdichter
- HD: Hochdruckbereich
- MD: Mitteldruckbereich
- ND: Niederdruckbereich
- R: Reihe
- S: Spur
- d: Abstand

## Patentansprüche

1. Kontinuierlich arbeitende Presse (1), umfassend
- zwei endlos umlaufende, Pressgut (8) durch die Presse (1) ziehende Stahlbänder (3), die zwischen sich zur Verpressung des Pressgutes (8) einen Pressspalt ausbilden,
- die Stahlbänder (3) abstützende temperierbare Heizplatten (10),
- eine zwischen den Stahlbändern (3) und den Heizplatten (10) angeordnete Wälzlagerabstützung,
- mehrere quer zur Umlaufrichtung (6) der Stahlbänder (3) angeordnete Gestelle (2) zur Aufnahme der Presskräfte, die über mehrere in einer Reihe (R) angeordnete hydraulische Arbeitszylinder (4) je Gestell zur Verpressung des Pressguts (8) zu einer Werkstoffplatte (11) und zur Einstellung des Pressspaltes direkt in die Heizplatte (10) eingeleitet werden, und
- ein Steuersystem (20) zur Steuerung und/oder Regelung des Betriebs der Presse (1),
**dadurch gekennzeichnet, dass** eine Messeinrichtung (12) zur Erfassung des Rohdichteprofils über die Plattendicke der Werkstoffplatte (11) vorgesehen ist, das Steuersystem (20) eingerichtet ist, die Arbeitszylinder (4) entlang der Presse gemäß einer Presskurve zu steuern und/oder zu regeln, und
das Steuersystem (20) eine Analysevorrichtung (23) umfasst, welche eingerichtet ist, die von der Messeinrichtung (12) erfassten Messwerte zu empfangen, das erfasste Rohdichteprofil, als ein Ist-Rohdichteprofil, hinsichtlich Abweichungen zu einem Soll-Rohdichteprofil zu analysieren, und basierend auf der Analyse an der Presskurve entlang der Presse vorzunehmende Änderungen zu bestimmen, um das Ist-Rohdichteprofil dem Soll-Rohdichteprofil anzunähern oder anzugleichen.

2. Kontinuierlich arbeitende Presse (1) nach Anspruch 1, wobei zur Anzeige von Empfehlungen der von der Analysevorrichtung (23) bestimmten an der Presskurve vorzunehmenden Änderungen eine Anzeige- und Bedienvorrichtung (24) angeordnet ist, so dass ein Pressenbediener der Presse (1) entsprechende Einstellungsänderungen vornehmen kann.

3. Kontinuierlich arbeitende Presse (1) nach Anspruch 1, wobei das Steuersystem (20) geeignet ist, die Presskurve automatisch gemäß den von der Analysevorrichtung (23) bestimmten an der Presskurve vorzunehmenden Änderungen anzupassen.

4. Kontinuierlich arbeitende Presse (1) nach einem der vorstehenden Ansprüche, wobei die Analysevorrichtung (23) geeignet ist das Ist-Rohdichteprofil in eine Mehrzahl von Bereichen zu unterteilen, wobei sich jeweils benachbarte Bereiche teilweise überlappen, und wobei die Analyse des Ist-Rohdichteprofils hinsichtlich der Abweichungen zu dem Soll-Rohdichteprofil bereichsweise erfolgt.

5. Kontinuierlich arbeitende Presse (1) nach Anspruch 4, so angeordnet, dass das Ist-Rohdichteprofil in zumindest fünf Bereiche unterteilt wird, wobei die Bereiche zwei Außenbereichen, zwei Übergangsbereichen und einem Kernbereich entsprechen.

6. Kontinuierlich arbeitende Presse (1) nach Anspruch 5, so angeordnet, dass das Ist-Rohdichteprofil in neun Bereiche unterteilt wird, wobei die beiden Außenbereiche und Übergangsbereiche in je zwei Bereiche unterteilt werden.

7. Kontinuierlich arbeitende Presse (1) nach einem der Ansprüche 4 bis 6, wobei der Außenbereich den Bereich mit einer mittleren Dichte größer 100%, bevorzugt größer 105% umfasst und/oder der Übergangsbereich in einem Bereich von 88% bis 110%, bevorzugt von 90% bis 108% der mittleren Dichte angeordnet ist und/oder der Kernbereich in einem Bereich von 75% bis 95%, bevorzugt von 80% bis 93% der mittleren Dichte angeordnet ist.

8. Kontinuierlich arbeitende Presse (1) nach einem der vorstehenden Ansprüche, wobei die Analysevorrichtung (23) als eine Fuzzy- Regeleinheit ausgebildet ist oder diese umfasst.

9. Kontinuierlich arbeitende Presse (1) nach einem der vorstehenden Ansprüche, wobei die Analysevorrichtung (23) als ein neuronales Netz, insbesondere ein Deep Learning-neuronales Netz ausgebildet ist oder dieses umfasst.

10. Kontinuierlich arbeitende Presse (1) nach einem der vorstehenden Ansprüche, wobei die Analysevorrichtung (23) ausgebildet ist, als Eingabe zusätzlich zumindest einen und bevorzugt alle der folgenden weiteren Parameter und/oder Messwerte zu berücksichtigen: Holzart, Faserlänge, Faserqualität, Kochzeit, Kochdruck, Mahlscheiben-Distanz, Bindemitteltyp, Bindemittelart, Bindemittelmenge, Härtertyp, Härterart, Härtermenge, Blasventilposition, Materialfeuchte, Schüttdichte des Materials, Fasertemperatur, Vorpressendruck, Pressfaktor der Presse, Mattenbefeuchtung / Besprühung, Matten-Flächengewicht, Mattenbreite, Mattentemperatur, Heizplattentemperatur, und Pressengeschwindigkeit.

11. Kontinuierlich arbeitende Presse (1) nach einem der vorstehenden Ansprüche, wobei das Steuersystem (20) weiter eingerichtet ist, kontinuierlich oder in regelmäßigen Abständen die gemessene Ist-Rohdichteprofile, die aktuelle Presskurve, sowie zusätzlich zumindest einen und bevorzugt alle der folgenden weiteren Parameter und/oder Messwerte zu berücksichtigen: Holzart, Faserlänge, Faserqualität, Kochzeit, Kochdruck, Mahlscheiben-Distanz, Bindemitteltyp, Bindemittelart, Bindemittelmenge, Härtertyp, Härterart, Härtermenge, Blasventilposition, Materialfeuchte, Schüttdichte des Materials, Fasertemperatur, Vorpressendruck, Pressfaktor der Presse, Mattenbefeuchtung / Besprühung, Matten-Flächengewicht, Mattenbreite, Mattentemperatur, Heizplattentemperatur, und Pressengeschwindigkeit.

12. Kontinuierlich arbeitende Presse (1) nach einem der vorstehenden Ansprüche, wobei die Analysevorrichtung (23) ausgebildet ist, als Eingabe zusätzlich zumindest einen und bevorzugt alle der folgenden weiteren Parameter und/oder Messwerte zu berücksichtigen: Partikelgröße, Partikelgrößenverteilung, Art der Zerkleinerungsvorrichtung, Standzeit der Zerkleinerungsvorrichtung und Schleifzugabe.

13. Kontinuierlich arbeitende Presse (1) nach einem der vorstehenden Ansprüche, wobei das Steuersystem (20) weiter eingerichtet ist, kontinuierlich oder in regelmäßigen Abständen die gemessene Ist-Rohdichteprofile, die aktuelle Presskurve, sowie zusätzlich zumindest einen und bevorzugt alle der folgenden weiteren Parameter und/oder Messwerte zu berücksichtigen: Partikelgröße, Partikelgrößenverteilung, Art der Zerkleinerungsvorrichtung, Standzeit der Zerkleinerungsvorrichtung und Schleifzugabe.

## Claims

1. Continuously operating press (1), comprising
- two endlessly circulating steel belts (3) drawing pressed material (8) through the press (1), which form a pressing gap between them for pressing the pressed material (8),
- temperature-controlled heating plates (10) supporting the steel belts (3),
- a roller bearing support arranged between the steel belts (3) and the heating plates (10),
- a plurality of frames (2) arranged transversely to the direction of rotation (6) of the steel belts (3) for absorbing the pressing forces, which are inserted via a plurality of hydraulic working cylinders (4) arranged in a row (R) per frame for pressing the pressed material (8) into a material board (11) and for setting the pressing gap directly into the heating plate (10), and
- a control system (20) for controlling and/or regulating the operation of the press (1),
**characterized in that** a measuring device (12) is provided for detecting the raw density profile via the board thickness of the material board (11), the control system (20) is set up to control and/or regulate the working cylinders (4) along the press in accordance with a press curve, and
the control system (20) comprises an analyzing device (23) which is set up to receive the measured values recorded by the measuring device (12), to analyze the recorded raw density profile, as an actual raw density profile, with regard to deviations from a target raw density profile, and to determine, based on the analysis, changes to be made to the press curve along the press in order to approximate or match the actual raw density profile to the target raw density profile.

2. Continuously operating press (1) according to claim 1, wherein a display and operating device (24) is arranged to display recommendations of the changes to be made to the press curve determined by the analyzing device (23), so that a press operator of the press (1) can make corresponding changes to the settings.

3. Continuously operating press (1) according to claim 1, wherein the control system (20) is suitable for automatically adjusting the press curve in accordance with the changes to be made to the press curve determined by the analyzing device (23).

4. Continuously operating press (1) according to one of the preceding claims, wherein the analyzing device (23) is adapted to divide the actual raw density profile into a plurality of ranges, wherein respectively adjacent ranges partially overlap, and wherein the analysis of the actual raw density profile with respect to the deviations from the target raw density profile is performed range by range.

5. Continuously operating press (1) according to claim 4, arranged such that the actual raw density profile is subdivided into at least five ranges, wherein the ranges correspond to two outer ranges, two transition ranges and a core range.

6. Continuously operating press (1) according to claim 5, arranged such that the actual raw density profile is divided into nine ranges, wherein the two outer ranges and transition ranges are each divided into two ranges.

7. Continuously operating press (1) according to one of claims 4 to 6, wherein the outer range comprises the range with an average density greater than 100%, preferably greater than 105%, and/or the transition range is arranged in a range from 88% to 110%, preferably from 90% to 108% of the average density, and/or the core range is arranged in a range from 75% to 95%, preferably from 80% to 93% of the average density.

8. Continuously operating press (1) according to one of the preceding claims, wherein the analyzing device (23) is designed as a fuzzy control unit or comprises the same.

9. Continuously operating press (1) according to one of the preceding claims, wherein the analyzing device (23) is designed as or comprises a neural network, in particular a deep learning neural network.

10. Continuously operating press (1) according to one of the preceding claims, wherein the analyzing device (23) is designed to additionally take into account as input at least one and preferably all of the following further parameters and/or measured values:
wood type, fiber length, fiber quality, cooking time, cooking pressure, grinding wheel distance, binder type, binder species, binder quantity, hardener type, hardener species, hardener quantity, blow valve position, material moisture, bulk density of the material, fiber temperature, pre-press pressure, press factor of the press, mat moistening/spraying, mat area weight, mat width, mat temperature, heating plate temperature, and press speed.

11. Continuously operating press (1) according to one of the preceding claims, wherein the control system (20) is further set up to continuously or at regular intervals take into account the measured actual raw density profiles, the current press curve, and additionally at least one and preferably all of the following further parameters and/or measured values:
wood type, fiber length, fiber quality, cooking time, cooking pressure, grinding wheel distance, binder type, binder species, binder quantity, hardener type, hardener species, hardener quantity, blow valve position, material moisture, bulk density of the material, fiber temperature, pre-press pressure, press factor of the press, mat moistening/spraying, mat area weight, mat width, mat temperature, heating plate temperature, and press speed.

12. Continuously operating press (1) according to one of the preceding claims, wherein the analyzing device (23) is designed to additionally take into account as input at least one and preferably all of the following further parameters and/or measured values:
particle size, particle size distribution, type of comminution device, service life of the comminution device and grinding addition.

13. Continuously operating press (1) according to one of the preceding claims, wherein the control system (20) is further set up to continuously or at regular intervals take into account the measured actual raw density profiles, the current press curve, as well as additionally at least one and preferably all of the following further parameters and/or measured values: particle size, particle size distribution, type of comminution device, service life of the comminution device and grinding addition.

## Revendications

1. Presse en continu (1), comprenant
- deux bandes d'acier (3) tournant sans fin et entraînant de la matière à presser (8) à travers la presse (1), qui forment une fente de pressage entre elles pour comprimer la matière à presser (8),
- des plaques chauffantes (10) pouvant être amenées à température et supportant les bandes d'acier (3),
- un appui sur roulement à billes disposé entre les bandes d'acier (3) et les plaques chauffantes (10),
- plusieurs châssis (2) disposés transversalement par rapport au sens de circulation (6) des bandes d'acier (3) pour absorber les forces de pressage qui sont exercées directement dans la plaque chauffante (10) par plusieurs vérins de travail hydrauliques (4) disposés en une rangée (R) pour chaque châssis pour comprimer la matière à presser (8) et produire un panneau de matériau (11) et pour ajuster la fente de pressage, et
- un système de commande (20) pour commander et/ou réguler le fonctionnement de la presse (1),
**caractérisée en ce qu'**il est prévu une installation de mesure (12) pour détecter le profil de masse volumique apparente sur l'épaisseur du panneau de matériau (11),
le système de commande (20) est configuré pour commander et/ou réguler les vérins de travail (4) le long de la presse selon une courbe de pressage
et le système de commande (20) comprend un dispositif d'analyse (23) configuré pour recevoir les valeurs de mesure captées par l'installation de mesure (12), analyser le profil de masse volumique apparente capté, qui représente un profil de masse volumique apparente réel, en termes d'écarts par rapport à un profil de masse volumique apparente de consigne et, sur la base de l'analyse, déterminer des changements à apporter à la courbe de pressage le long de la presse pour rapprocher ou aligner le profil de masse volumique apparente réel sur le profil de masse volumique apparente de consigne.

2. Presse en continu (1) selon la revendication 1, dans laquelle un dispositif d'affichage et de commande (24) est disposé pour afficher des recommandations des modifications à apporter à la courbe de pressage déterminés par le dispositif d'analyse (23), afin qu'un opérateur de la presse (1) puisse apporter les modifications de réglages correspondantes.

3. Presse en continu (1) selon la revendication 1, dans laquelle le système de commande (20) convient adapté pour ajuster automatiquement la courbe de pressage selon les changements à apporter à la courbe de pressage déterminés par le dispositif d'analyse (23).

4. Presse en continu (1) selon l'une des revendications précédentes, dans laquelle le dispositif d'analyse (23) convient pour diviser le profil de masse volumique apparente réel en plusieurs zones, dont les zones voisines se recoupent partiellement, et dans laquelle l'analyse du profil de masse volumique apparente réel en termes d'écarts par rapport au profil de masse volumique apparente de consigne est effectuée par zones.

5. Presse en continu (1) selon la revendication 4, disposée de telle manière que le profil de masse volumique apparente réel est divisé en au moins cinq zones, dans laquelle les zones correspondent à deux zones extérieures, deux zones de transition et une zone centrale.

6. Presse en continu (1) selon la revendication 5, disposée de telle manière que le profil de masse volumique apparente réel est divisé en neuf zones, dans laquelle les deux zones extérieures et zones de transition sont divisées chacune en deux zones.

7. Presse en continu (1) selon l'une des revendications 4 à 6, dans laquelle la zone extérieure comprend la zone ayant une masse volumique moyenne supérieure à 100 % de la masse volumique moyenne, de préférence supérieure à 105 % et/ou la zone de transition est disposée dans une zone de 88 % à 110 %, de préférence de 90 % à 108 % de la masse volumique moyenne et/ou la zone centrale est disposée dans une zone de 75 % à 95 %, de préférence de 80 % à 93 %.

8. Presse en continu (1) selon l'une des revendications précédentes, dans laquelle le dispositif d'analyse (23) est conçu comme une unité de régulation à logique floue ou contient celle-ci.

9. Presse en continu (1) selon l'une des revendications précédentes, dans laquelle le dispositif d'analyse (23) est conçu comme un réseau neuronal, en particulier un réseau neuronal d'apprentissage profond, ou contient celui-ci.

10. Presse en continu (1) selon l'une des revendications précédentes, dans laquelle le dispositif d'analyse (23) est conçu pour prendre en outre en compte à l'entrée au moins un des autres paramètres et/ou valeurs de mesure suivants, et de préférence tous : type de bois, longueur des fibres, qualité des fibres, temps de cuisson, pression de cuisson, distance entre les meules, type de liant, nature du liant, quantité de liant, type de durcisseur, nature du durcisseur, quantité de durcisseur, position des soupapes de soufflage, humidité du matériau, masse volumique apparente du matériau, température des fibres, pression de prépresse, facteur de pressage de la presse, humidification/arrosage de la nappe, poids par unité de surface de la nappe, largeur de la nappe, température de la nappe, température des plaques chauffantes et vitesse de la presse.

11. Presse en continu (1) selon l'une des revendications précédentes, dans laquelle le système de commande (20) est en outre configuré pour prendre en compte en continu ou à intervalles réguliers les profils de masse volumique apparente réels, la courbe de pressage instantanée et, en outre, au moins un des autres paramètres et/ou valeurs de mesure suivants, et de préférence tous : type de bois, longueur des fibres, qualité des fibres, temps de cuisson, pression de cuisson, distance entre les meules, type de liant, nature du liant, quantité de liant, type de durcisseur, nature du durcisseur, quantité de durcisseur, position des soupapes de soufflage, humidité du matériau, masse volumique apparente du matériau, température des fibres, pression de prépresse, facteur de pressage de la presse, humidification/arrosage de la nappe, poids par unité de surface de la nappe, largeur de la nappe, température de la nappe, température des plaques chauffantes et vitesse de la presse.

12. Presse en continu (1) selon l'une des revendications précédentes, dans laquelle le dispositif d'analyse (23) est configuré pour prendre en outre en compte à l'entrée au moins un des autres paramètres et/ou valeurs de mesure suivants, et de préférence tous : grosseur des particules, répartition granulométrique des particules, nature du dispositif de meulage, temps de passage dans le dispositif de meulage et surépaisseur pour meulage.

13. Presse en continu (1) selon l'une des revendications précédentes, dans laquelle le système de commande (20) est en outre configuré pour prendre en compte en continu ou à intervalles réguliers les profils de masse volumique apparente réels mesurés, la courbe de pressage instantanée et, en outre, au moins un des autres paramètres et/ou valeurs de mesure suivants, et de préférence tous : grosseur des particules, répartition granulométrique des particules, nature du dispositif de meulage, temps de passage dans le dispositif de meulage et surépaisseur pour meulage.
